(19) Europäisches Patentamt
European Patent Office
Office européen des brevets

(11) Veröffentlichungsnummer: **0 322 692 B1**

(12) # EUROPÄISCHE PATENTSCHRIFT

(45) Veröffentlichungstag der Patentschrift: **06.03.91 Patentblatt 91/10**

(51) Int. Cl.$^5$: **C07D 307/85**, C07D 333/70, C07D 209/42, A61K 31/34, A61K 31/38, A61K 31/40

(21) Anmeldenummer: **88121188.2**

(22) Anmeldetag: **17.12.88**

(54) **Heterocyclisch substituierte Alkylsulfonamide, Verfahren zu ihrer Herstellung sowie Arzneimittel.**

Die Akte enthält technische Angaben, die nach dem Eingang der Anmeldung eingereicht wurden und die nicht in dieser Patentschrift enthalten sind.

(30) Priorität: **24.12.87 DE 3744141**
**15.02.88 DE 3804636**

(43) Veröffentlichungstag der Anmeldung: **05.07.89 Patentblatt 89/27**

(45) Bekanntmachung des Hinweises auf die Patenterteilung: **06.03.91 Patentblatt 91/10**

(84) Benannte Vertragsstaaten: **AT BE CH DE ES FR GB GR IT LI LU NL SE**

(56) Entgegenhaltungen:
**EP-A- 0 146 243**
**EP-A- 0 173 899**

(56) Entgegenhaltungen:
**Chemical Abstracts, Band 80, Nr 1,7. Jänner 1974, Columbus, Ohio, U.S.A. Bailey, A. Sidney et al. "Examination of the reactions of simple indoles with arenesulfonyl azides" Seite 291, Spalte 2, Zusammenfassung Nr 3 337c**

(73) Patentinhaber: **BOEHRINGER MANNHEIM GMBH**
**Sandhofer Strasse 116**
**W-6800 Mannheim 31 (DE)**

(72) Erfinder: **Friebe, Walter-Gunar, Dr.rer.nat.**
**Sophienstrasse 8**
**W-6800 Mannheim 1 (DE)**
Erfinder: **Reinholz, Erhard, Dr.**
**Kussmaulstrasse 9**
**W-6800 Mannheim 1 (DE)**
Erfinder: **Doerge, Liesel, Dr.med.**
**Schwalbenstrasse 26**
**W-6840 Lampertheim (DE)**
Erfinder: **Stegmeier, Karlheinz, Dr.rer.nat.**
**Kirchbergstrasse 17**
**W-6148 Heppenheim (DE)**

EP 0 322 692 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Die vorliegende Erfindung betrifft neue Sulfonamide der allgemeinen formel I,

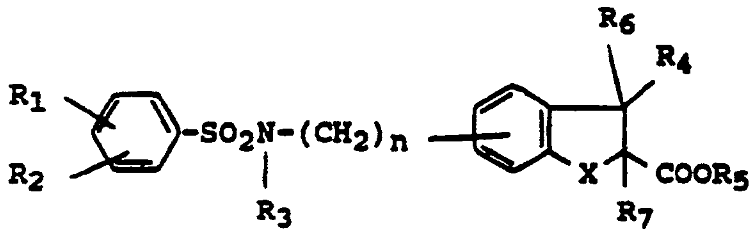

(I),

in welcher

$R_1$ und $R_2$ gleich oder verschieden sein können und jeweils Wasserstoff, ein Halogenatom, eine $C_1$- bis $C_6$-Alkyl-, $C_1$- bis $C_6$-Alkoxy-, $C_1$- bis $C_6$-Alkylthio-, $C_1$- bis $C_6$-Alkylsulfinyl-, $C_1$- bis $C_6$-Alkylsulfonyl-, Trifluormethyl-, Hydroxy- oder Cyanogruppe bedeuten,

$R_3$ und $R_8$ gleich oder verschieden sein können und jeweils Wasserstoff, eine $C_1$- bis $C_6$-Alkyl-, eine Benzyl- oder eine $C_1$- bis $C_6$-Alkanoylgruppe bedeuten,

$R_4$ und $R_5$ gleich oder verschieden sein können und jeweils Wasserstoff oder eine $C_1$- bis $C_6$-Alkylgruppe bedeuten,

$R_6$ und $R_7$ jeweils Wasserstoff darstellen oder gemeinsam eine Bindung bilden,

X für Sauerstoff, Schwefel, eine Gruppe NOH oder eine Gruppe $NR_8$ steht und

n eine ganze Zahl von 1 bis 4 bedeutet,

deren physiologisch verträgliche Salze anorganischer oder organischer Basen und Verfahren zu ihrer Herstellung sowie diese Verbindungen enthaltende Arzneimittel.

Für den Fall, daß die Verbindungen der allgemeinen Formel I ein asymmetrisches Kohlenstoffatom enthalten, sind auch die optisch aktiven Verbindungen und racemischen Gemische Gegenstand der Erfindung.

In der EP-A-0 146 243 sind struktur analoge verbindungen beschrieben, die als lipoxygenase-inhibitonen verwendung finden.

Die neuen Verbindungen der allgemeinen Formel I weisen wertvolle pharmakologische Eigenschaften auf, insbesondere zeigen sie eine ausgezeichnete antagonistische Wirkung gegenüber Thromboxan $A_2$ sowie gegen Prostaglandin-endo-peroxide. Sie inhibieren die Aggregation von Blutplättchen und verhindern die Konstriktion der glatten Muskulatur sowie die Bronchokonstriktion. Sie sind außerdem wertvolle Heilmittel zur Behandlung pathologischer Veränderungen der Nierenfunktion.

Diese Eigenschaften machen sie zu wertvollen Heilmitteln zur Behandlung beispielweise von cardiovaskulären Erkrankungen und von Asthma und zur Prophylaxe einer Schocklunge. Sie können weiterhin verwendet werden bei Organtransplantationen und Nierendialyse und sind geeignet, Rezidive bei Magengeschwüren zu verhindern. Eine besondere Bedeutung liegt in der Möglichkeit, thrombotische Prozesse günstig zu beeinflussen oder zu verhindern. Sie sind zur Behandlung peripherer arterieller Verschlußkrankheiten geeignet und können beispielsweise gegen cerebrale und ischaemische Zustände eingesetzt werden.

Stehen $R_1$, $R_2$, $R_3$, $R_4$, $R_5$ oder $R_8$ für Alkyl-, Alkoxy-, Alkylthio-, Alkylsulfinyl-, Alkylsulfonyl- oder Alkanoylgruppen, so können deren Alkylbestandteile geradkettig oder verzweigt sein. Bevorzugt sind Methyl-, Ethyl-, Propyl-, Isopropyl-, Butyl-, sek.-Butyl-, tert.-Butyl-, Pentyl- und Hexylreste.

Als Halogenatome kommen Fluor, Chlor und Brom in Frage.

Bevorzugte Verbindungen der vorliegenden Erfindung sind Verbindungen der Formel I, in welcher

$R_1$, $R_3$ und $R_8$ Wasserstoff

$R_2$ Wasserstoff, Brom, Chlor, Methyl oder Methoxy

$R_4$ Wasserstoff oder Methyl

$R_5$ Wasserstoff oder Ethyl bedeuten,

$R_6$ und $R_7$ gemeinsam eine Bindung bilden,

X für Sauerstoff, Schwefel oder eine Gruppe $NR_8$ steht,

n eine ganze Zahl 1 oder 2 bedeutet und der Sulfonamidorest in 5-Stellung des Heterocyclus steht.

Das erfindungsgemäße Verfahren zur Herstellung der Verbindungen der Formel I ist dadurch gekennzeichnet, daß man in an sich bekannter Weise entweder

a) eine Verbindung der allgemeinen Formel II

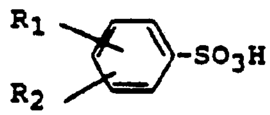

(II),

in welcher
$R_1$ und $R_2$ die obengenannte Bedeutung haben, oder einem reaktiven Derivat hiervon, mit einer Verbindung der allgemeinen Formel III

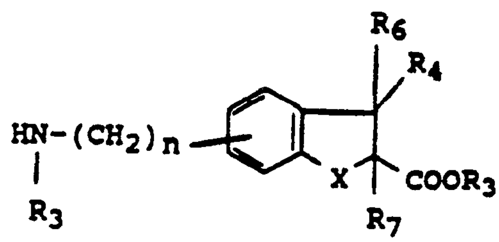

(III),

in welcher
$R_3$, $R_4$, $R_5$, $R_5$, $R_7$, X und n die obengenannte Bedeutung haben, oder
b) eine Verbindung der allgemeinen Formel IV

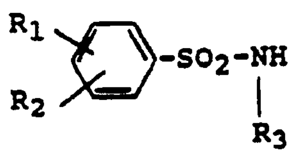

(IV),

in welcher
$R_1$, $R_2$ und $R_3$ die obengenannte Bedeutung haben, mit einer Verbindung der allgemeinen Formel V

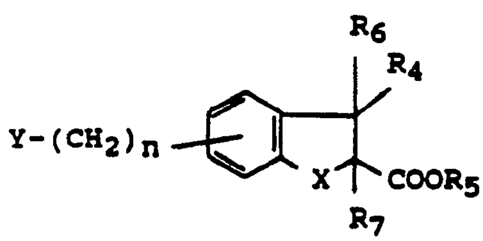

(V),

in welcher
$R_4$, $R_5$, $R_6$, $R_7$, X und n die obengenannte Bedeutung haben und Y einen reaktiven Rest darstellt, umsetzt, oder
c) für den Fall, daß X die Gruppe NH darstellt und $R_6$ und $R_7$ gemeinsam eine Bindung bilden, gemäß einer kombinierten Japp-Klingemann/Fischer-Indol-Synthese eine Verbindung der Formel VI

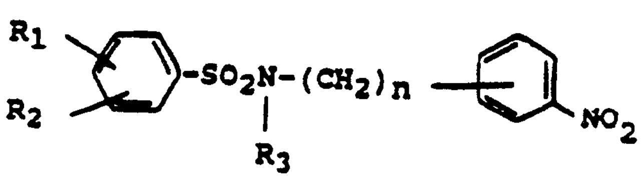

(VI)

in der $R_1$, $R_2$, $R_3$ und n die angegebene Bedeutung haben, reduziert, die entstandene Aminogruppe in üblicher Weise in eine Diazoniumgruppe überführt, die Diazoniumgruppe mit 2-Methylacetessigester umsetzt und das entstandene Hydrazon zum Indol cyclisiert, und anschließend gewünschtenfalls einen Rest $R_3$, $R_5$ oder $R_8$ in einen anderen, durch die jeweilige Definition von $R_3$, $R_5$ oder $R_8$ gegebenen Rest umwandelt, eine von $R_6$ und $R_7$ gemeinsam gebildete Bindung durch Hydrogenolyse aufhebt, eine für

$R_1$ oder $R_2$ stehende Alkylthiogruppe durch Oxidation in eine Alkylsulfinylgruppe oder Alkylsulfonylgruppe überführt, eine für $R_1$ oder $R_2$ stehende Alkoxygruppe in eine Hydroxygruppe oder eine Hydroxygruppe in eine Alkoxygruppe umwandelt und die erhaltenen Verbindungen der Formel I gewünschtenfalls durch Neutralisation mit nichttoxischen Basen in ihre physiologisch verträglichen Salze umwandelt.

Die Umsetzung einer Verbindung der Formel II mit einer Verbindung der Formel III wird zweckmäßig in einem Lösungsmittel oder Lösungsmittelgemisch wie Dichlormethan, Ether, Tetrahydrofuran, Dioxan oder Benzol gegebenenfalls in Gegenwart eines säurebindenden Mittels wie Natriumcarbonat, Triethylamin oder Pyridin, wobei die beiden letzteren gleichzeitig auch als Lösungsmittel dienen können, in Gegenwart eines die Säure aktivierenden oder wasserentziehenden Mittels wie Thionylchlorid oder Phosphorpentachlorid, vorzugsweise jedoch mit einem reaktionsfähigen Derivat einer Verbindung der Formel II, beispielsweise mit deren Anhydrid oder Halogenid, vorzugsweise bei Temperaturen zwischen 0 und 100°C, zum Beispiel bei Temperaturen zwischen Raumtemperatur und 50°C, durchgeführt.

Für einen reaktiven Rest Y stehen vorzugsweise Halogenatome wie Chlor oder Brom oder aliphatische oder aromatische Sulfonyloxygruppen wie beispielsweise Methansulfonyloxy oder p-Toluolsulfonyloxy.

Die Umsetzung einer Verbindung der Formel IV mit einer Verbindung der Formel V wird zweckmäßig in einem Lösungsmittel wie Aceton, Ether, Benzol, Toluol oder Dimethylformamid bei Temperaturen zwischen –30 und 100°C, vorzugsweise bei Raumtemperatur in Gegenwart einer Base wie Kaliumcarbonat oder Natriumhydrid oder auch in einem Alkohol in Gegenward eines Alkalialkoholats vorgenommen.

Die Verbindungen der Formel III sind größtenteils neu und können nach literaturbekannten Verfahren aus beschriebenen Vorstufen synthetisiert werden. So werden z.B. Halogenmethyl-Derivate von Benzofuran-, Benzothiophen- bzw. Indol-2-carbonsäure mit Cyaniden zu den entsprechenden Cyanmethylverbindungen umgesetzt, die anschließend zu den Aminoethylderivaten reduziert werden. Verbindungen der Formel III mit n = 1 werden direkt aus dem Halogenmethyl-Derivaten durch Umsetzung mit $R_3NH_2$ erhalten.

Die Halogenmethyl-Derivate der Formel V (n = 1, Y = Cl) werden durch Chlormethylierung der entsprechenden Heterocyclen erhalten.

Die Umwandlung von Verbindungen der allgemeinen Formel I in andere Verbindungen der allgemeinen formel I kann nach geläufigen Methoden erfolgen.

Steht für $R_3$ oder $R_8$ beispielsweise ein Wasserstoffatom, so kann dieses durch Umsetzung mit einem Alkylhalogenid, Alkylsulfonat oder Alkylsulfat durch einen Alkylrest, mit einer Carbonsäure oder einem aktivierten Carbonsäurederivat wie Anhydrid, Halogenid oder Ester durch einen Alkanoylrest substituiert werden.

Ein für $R_3$ oder $R_8$ stehender Alkanoylrest kann durch saure oder alkalische Hydrolyse oder Alkoholyse durch ein Wasserstoffatom ersetzt werden.

Die Umwandlung eines für $R_5$ stehenden Restes erfolgt beispeilsweise durch Verseifung, Veresterung oder Umesterung.

Die Hydrogenolyse einer durch $R_6$ und $R_7$ gemeinsam gebildeten Bindung führt man zweckmäßig in einem Lösungsmittel wie Wasser, wässriges Ethanol, Methanol, Essigsäure, Essigester, Tetrahydrofuran oder Dimethylformamid mit Wasserstoff oder Hydrazin in Gegenwart eines Hydrierkatalysators wie Raney-Nickel, Platin oder Palladium-Kohle durch.

Die Oxidation einer für $R_1$ oder $R_2$ stehenden Alkylthiogruppe nimmt man vorzugsweise in einem Lösungsmittel oder Lösungsmittelgemisch wie beispielsweise Wasser, Alkohol, wässriges Pyridin, Aceton, Essigsäure, verdünnte Schwefelsäure oder Trifluoressigsäure bei Temperaturen zwischen –80 und 100°C vor.

Zur Herstellung einer Alkylsulfinylgruppe wird die Oxidation zweckmäßig mit einem Äquivalent des verwendeten Oxidationsmittels durchgeführt, beispielsweise mit Wasserstoffperoxid in Eisessig, Trifluoressigsäure oder Ameisensäure bei 0 bis 20°C oder in Aceton bei 0 bis 60°C, mit einer Persäure wie Perameisensäure in Eisessig oder Trifluoressigsäure bei 0 bis 50°C oder mit m-Chlorperbenzoesäure in Dichlormethan oder Chloroform bei –20 bis 60°C, mit Natriummetaperiodat in wässrigem Methanol oder Ethanol bei –15 bis 25°C, mit Brom in Eisessig oder wässriger Essigsäure, mit N-Bromsuccinimid in Ethanol, mit tert.-Butylhypochlorit in Methanol bei –80 bis –30°C, mit Jodbenzodichlorid in wässrigem Pyridin bei 0 bis 50°C, mit Salpetersäure in Eisessig bei 0 bis 20°C, mit Chromsäure in Eisessig oder in Aceton bei 0 bis 20°C oder mit Sulfurylchlorid in Dichlormethan bei –70°C ; der hierbei erhaltene Thioether-Chlor-Komplex wird zweckmäßig mit wässrigem Ethanol hydrolysiert.

Zur Herstellung einer Alkylsulfonylgruppe wird die Oxidation zweckmäßig mit zwei oder mehr Aequivalenten des verwendeten Oxidationsmittels durchgeführt, beispielsweise mit den vorgenannten Reagenzien bei gewünschtenfalls höherer Temperatur bis 100°C.

Die Umwandlung einer für $R_1$ oder $R_2$ stehenden Alkoxygruppe in eine Hydroxygruppe wird entweder

in Gegenwart einer Säure wie Bromwasserstoffsäure oder Iodwasserstoffsäure, einer Lösung von Bromwasserstoff in Eisessig oder Chlorwasserstoff in Pyridin oder in Gegenwart einer Lewissäure wie Aluminiumtrichlorid, Bortrichlorid oder Bortribromid in einem Lösungsmittel wie Dichlormethan bei Temperaturen zwischen −80 und 100°C durchgeführt.

Für die Alkylierung einer für $R_1$ oder $R_2$ stehenden Hydroxygruppe setzt man vorzugsweise in einem Lösungsmittel wie Aceton, Alkohol, Ether, Benzol, Toluol oder Dimethylformamid bei Temperaturen zwischen −30 und 100°C, vorzugsweise bei Raumtemperatur in Gegenwart einer Base wie Kaliumcarbonat, Natriumhydrid oder einem Alkalialkoholat eine Alkylierungsmittel wie Alkylhalogenid, Alkylsulfat oder Alkylsulfonat ein.

Zur Herstellung von Salzen mit physiologisch verträglichen organischen oder anorganischen Basen wie beispielsweise Natriumhydroxid, Kaliumhydroxid, Calciumhydroxid, Ammoniumhydroxid, Methylglucamin, Morpholin, Triethylamin oder Ethanolamin können die Verbindungen der Formel I, in denen mindestens einer der Reste $R_3$ oder $R_6$ Wasserstoff bedeutet, mit den entsprechenden Basen umgesetzt werden. Auch Mischungen der sauren Verbindungen mit einem geeigneten Alkalicarbonat bzw. -hydrogencarbonat kommen in Betracht.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z.B. Tartrat- oder Borat-Puffer, Ethanol, Dimethylsulfoxid, Komplexbildner (wie Ethylendiamintetraessigsäure), hochmolekulare Polymere (wie flüssiges Polyethylenoxid) zur Viskositätsregulierung oder Polyethylen-Derivate von Sorbitanhydriden. Feste Trägerstoffe sind z.B. Stärke, Lactose, Mannit, Methylcellulose, Talkum, hochdisperse Kieselsäure, höhermolekulare Fettsäuren (wie Stearinsäure), Gelatine, Agar-Agar, Calciumphosphat, Magnesiumstearat, tierische und pflanzliche Fette oder feste hochmolekulare Polymere (wie Polyethylenglykole). Für die orale Applikation geeignete Zubereitungen können gewünschtenfalls Geschmacks- und Süßstoffe enthalten.

Die verabreichte Dosierung hängt vom Alter, der Gesundheit und dem Gewicht des Empfängers, dem Ausmaß der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis der aktiven Verbindung 0,1 bis 50 mg/kg Körpergewicht. Normalerweise sind 0,5 bis 40 und vorzugsweise 1,0 bis 20 mg/kg in einer oder mehreren Anwendungen pro Tag wirksam, um die gewünschten Resultate zu erhalten.

Bevorzugt im Sinne der Erfindung sind außer den in den Beispielen genannten Verbindungen der Formel I sowie deren Salzen insbesondere die folgenden :

5-[2-(3-Trifluormethyl-benzolsulfonamido)ethyl]benzo[b] furan-2-carbonsäure
5-[2-(4-Cyan-benzolsulfonamido)ethyl]benzo[b]furan-2-carbonsäure
5-[2-(4-Methylthio-benzolsulfonamido)ethyl]benzo[b]furan-2-carbonsäure
5-[2-(4-Methylsulfinyl-benzolsulfonamido)ethyl]benzo[b] furan-2-carbonsäure
5-[2-(4-Methylsulfonyl-benzolsulfonamido)ethyl]benzo[b] furan-2-carbonsäure
5-[2-(4-Hydroxy-benzolsulfonamido)ethyl]benzo[b]furan-2-carbonsäure
5-[2-(2,4-Dimethyl-benzolsulfonamido)ethyl]benzo[b]furan-2-carbonsäure
5-[2-(N-Benzyl-4-chlor-benzolsulfonamido)ethyl]benzo[b] furan-2-carbonsäure
5-[2-(4-N-Acetyl-4-chlor-benzolsulfonamido)ethyl]benzo[b] furan-2-carbonsäure
5-[3-(4-Chlor-benzolsulfonamido)propyl]benzo[b]furan-2-carbonsäure
5-[4-(4-Chlor-benzolsulfonamido)butyl]benzo[b]furan-2-carbonsäure
5-[2-(4-Chlor-benzolsulfonamido)ethyl]-N-acetyl-indol-2-carbonsäure
5-[2-(4-Chlor-benzolsulfonamido)ethyl]-N-benzoyl-indol-2-carbonsäure

Beispiel 1

5-[2-(4-Chlorbenzolsulfonamido)ethyl]benzo[b]furan-2-carbonsäureethylester

Eine Suspension von 8.1 g (30 mmol) 5-(2-Amino-ethyl)benzo[b]furan-2-carbonsäureethylester-hydrochlorid in 100 ml Dichlormethan versetzt man mit 10.0 g (100 mmol) Triethylamin, kühlt auf 5°C und tropft eine Lösung von 8.4 g (40 mmol) 4-Chlor-benzolsulfochlorid ein. Man rührt 4 h bei Raumtemperatur nach,

engt ein, nimmt in Ethylacetat auf, wäscht neutral, engt ein und chromatographiert an Kieselgel. Elution mit Dichlormethan ergibt 5.5 g Titelverbindung (45% d.Th.) vom Schmp. 123-125°C.

Das Ausgangsmaterial kann wie folgt erhalten werden :

Durch Umsetzung von 5-Chlormethyl-benzo[b]furan-2-carbonsäureethylester (Chem. Abstr. 1959, 3185 d) mit überschüssigem Natriumcyanid in Dimethylformamid gelangt man mit 90% Ausbeute zum 5-Cyanomethyl-benzo[b]furan-2-carbonsäureethylester vom Schmp. 74-76°C (aus Ethylacetat), welcher in essigsaurer Lösung über Platinoxid zum 5-(2-Amino-ethyl)benzo[b]furan-2-carbonsäureethylester hydriert wird. Aus dessen ethanolischer Lösung kann mit etherischer Chlorwasserstofflösung das Hydrochlorid vom Schmp. 188-190°C gefällt werden (Ausbeute 64% d.Th.).

Beispiel 2

In analoger Weise wie in Beispiel 1 beschrieben erhält man aus 5-(2-Amino-ethyl)benzo[b]furan-2-carbonsäureethylester-hydrochlorid und den angegebenen Sulfonsäurederivaten :

| | Bezeichnung | Ausb. % | Schmelzpunkt °C (Lsg.mittel) |
|---|---|---|---|
| a) | 5-(2-Benzolsulfonamido-ethyl)benzo[b]furan-2-carbonsäureethylester aus Benzolsulfochlorid | 41 | 84-86 (Essigester) |
| b) | 5-[2-(4-Methyl-benzolsulfon-amido)ethyl]benzo[b]furan-2-carbonsäureethylester aus p-Toluolsulfonsäureanhydrid | 49 | 101-103 (Essigester) |
| c) | 5-[2-(3-Methyl-benzolsulfon-amido)ethyl]benzo[b]furan-2-carbonsäureethylester aus 3-Methyl-benzolsulfochlorid | 57 | 86-88 (Dichlormethan) |
| d) | 5-[2-(4-Methoxy-benzolsulfon-amido)ethyl]benzo[b]furan-2-carbonsäureethylester aus 4-Methoxy-benzolsulfochlorid | 52 | 91-93 (Dichlormethan) |
| e) | 5-[2-(4-Brom-benzolsulfon-amido)ethyl]benzo[b]furan-2-carbonsäurethylester aus 4-Brom-benzolsulfochlorid | 51 | 132-135 (Essigester) |

Beispiel 3

5-[2-(4-Chlor-benzolsulfonamido)ethyl]benzo[b]thiophen-2-carbonsäureethylester

In analoger Weise wie in Beispiel 1 beschrieben erhält man die Titelverbindung aus 4-Chlor-benzolsulfochlorid und 5-(2-Aminoethyl)benzo[b]thiophen-2-carbonsäureethylester.

Beispiel 4

5-[2-(4-Chlor-benzolsulfonamido)ethyl]-3-methyl-benzo[b]furan-2-carbonsäureethylester

In analoger Weise wie in Beispiel 1 beschrieben erhält man die Titelverbindung aus 4-Chlor-benzolsulfochlorid und 5-(2-Aminoethyl)-3-methyl-benzo[b]furan-2-carbonsäureethylester.

Beispiel 5

5-(4-Chlor-benzolsulfonamido)methyl)benzo[b]furan-2-carbonsäureethylester

In analoger Weise wie in Beispiel 1 beschrieben erhält man die Titelverbindung aus 4-Chlor-benzolsulfochlorid und 5-Aminomethyl-benzo[b]furan-2-carbonsäureethylester.

Beispiel 6

6-[2-(4-Chlor-benzolsulfonamido)ethyl]benzo-[b]furan-2-carbonsäureethylester

In analoger Weise wie in Beispiel 1 beschriben erhält man die Titelverbindung aus 4-Chlor-benzolsulfochlorid und 6-(2-Aminoethyl)benzo[b]furan-2-carbonsäureethylester.

Beispiel 7

4-[2-Chlor-benzolsulfonamido)ethyl]benzo-[b]furan-2-carbonsäureethylester

In analoger Weise wie in Beispeil 1 beschrieben erhält man die Titelverbindung aus 4-Chlor-benzolsulfochlorid und 4-(2-Aminoethyl)benzo[b]furan-2-carbonsäureethylester.

Beispiel 8

5-[2-(4-Chlor-benzolsulfonamido)ethyl]-2,3-dihydro-benzo[b]furan-2-carbonsäureethylester

In analoger Weise wie in Beispiel 1 beschrieben erhält man die Titelverbindung aus 4-Chlor-benzolsulfochlorid und 5-(2-Aminoethyl)-2,3-dihydro-benzo[b]furan-2-carbonsäureethylester.

Beispiel 9

5-[2-(4-Chlor-benzolsulfonamido)ethyl]indol-2-carbonsäureethylester

In analoger Weise wie in Beispiel 1 beschrieben erhält man die Titelverbindung aus 4-Chlor-benzolsulfochlorid und 5-(2-Aminoethyl) indol-2-carbonsäureethylester (Ausbeute 34% ; Schmp. 193-195° C, aus Ethanol.

Beispiel 10

5-[2-(4-Chlor-benzolsulfonamido)ethyl]-N-hydroxy-indol-2-carbonsäureethylester

In analoger Weise wie in Beispiel 1 beschrieben erhält man die Titelverbindung aus 4-Chlor-benzolsulfochlorid und 5-(2-Aminoethyl)-N-hydroxy-indol-2-carbonsäureethylester.

Beispiel 10 a

5-[2-(4-tert-Butyl-benzolsulfonamido)ethyl]benzo[b]-furan-2-carbonsäureethylester

In analoger Weise wie in Beispiel 1 beschrieben erhält man die Titelverbindung aus 4-tert-Butyl-benzolsulfochlorid und 5-(2-Amino-ethyl)benzo[b]-furan-2-carbonsäureethylester.

Beispiel 11

5-[2-(4-Chlor-benzolsulfonamido)ethyl]benzo[b]furan-2-carbonsäure

Eine Mischung aus 5.0 g (12.2 mmol) Verbindung des Beispiels 1, 25 ml Ethanol und 25 ml 2 N Natronlauge rührt man 1 h bei 50°C, engt ein, wäscht mit Ethylacetat, stellt die wässrige Phase sauer, filtriert und

trocknet den Niederschlag im Vakuum. Man isoliert 4.3 g der Titelverbindung (93% d.Th.) vom Schmp. 193-195°C.

Beispiel 12

In analoger Weise wie in Beispiel 11 beschrieben erhält man :

| | Bezeichnung | Ausb. % | Schmelzpunkt °C (Lsg.mittel) |
|---|---|---|---|
| a) | 5-(2-Benzolsulfonamido-ethyl-benzo[b]furan-2-carbonsäure aus Verbindung des Beisp. 2a | 92 | 222-225 (Wasser) |
| b) | 5-[2-(4-Methyl-benzolsulfonamido)ethyl]benzo[b]furan-2-carbonsäure aus Verbindung des Beisp. 2b | 95 | 209-211 (Wasser) |
| c) | 5-[2-(3-Methyl-benzolsulfonamido)ethyl]benzo[b]furan-2-carbonsäure aus Verbindung des Beisp. 2c | 94 | 212-214 (Wasser) |
| d) | 5-[2-(4-Methoxy-benzolsulfonamido)ethyl]benzo[b]furan-2-carbonsäure aus Verbindung des Beisp. 2d | 85 | 194-196 (Wasser) |
| e) | 5-[2-(4-Brom-benzolsulfonamido)ethyl]benzo[b]furan-2-carbonsäure aus Verbindung des Beisp. 2e | 93 | 198-200 (Wasser) |
| f) | 5-[2-(4-Chlor-benzolsulfonamido)ethyl]benzo[b]thiophen-2-carbonsäure aus Verbindung des Beisp. 3 | | |
| g) | 5-[2-(4-Chlor-benzolsulfonamido)ethyl]-3-methyl-benzo-[b]furan-2-carbonsäure aus Verbindung des Beisp. 4 | 78 | 201-202 (Essigester) |
| h) | 5(4-Chlor-benzolsulfonamidomethyl)benzo-[b]furan-2-carbonsäure aus Verbindung des Beisp. 5 | 51 | 234-238 (Essigester) |
| i) | 6-[2-(4-Chlor-benzolsulfonamido)ethyl]benzo[b]furan-2-carbonsäure aus Verbindung des Beisp. 6 | | |
| j) | 4-[2-(4-Chlor-benzolsulfonamido)ethyl]benzo[b]furan-2-carbonsäure aus Verbindung des Beisp. 7 | | |

| | | | |
|---|---|---|---|
| k) | 5-[2-(4-Chlor-benzolsulfonamido)ethyl]-2,3-dihydro-benzo[b]furan-2-carbonsäure aus Verbindung des Beisp. 8 | | |
| l) | 5-[2-(4-Chlor-benzolsulfon-amido)ethyl]indol-2-carbon-säure aus Verbindung des Beisp. 9 | 72 | 236-237 (Eisessig) |
| m) | 5-[2-(4-Chlor-benzolsulfonamido)ethyl]-N-hydroxy-indol-2-carbonsäure aus Verbindung des Beisp. 10 | | |
| n) | 5-[2-(4-tert-Butyl-benzolsulfonamido)ethyl]benzo[b]-furan-2-carbonsäure aus Verbindung des Beisp. 10a | 80 | 193-196 (Ether) |

Beispiel 13

5-(2-Benzolsulfonamido-ethyl)-2,3-dihydrobenzo[b]furan-2-carbonsäure

Eine Mischung aus 1.2 g Verbindung des Beispiels 2a, 50 ml Ethanol und 0,5 g Palladiumkohle wird 3 Tage unter 280 bar Wasserstoffdruck auf 100°C erhitzt. Man versetzt mit verd. Natronlauge, erwärmt 2h auf 50°C, filtriert, säuert an, filtriert und reinigt den Niederschlag durch Chromatographie.

Man isoliert 300 mg (25% d.Th.) titelverbindung, vom Schmp. 132-133°C (aus Ether).

Beispiel 14

5-[2-(4-Chlor-benzolsulfonamido)ethyl)indol-2-carbonsäureethylester

Die Verbindung des Beispiels 9 kann auch auf folgendem Wege erhalten werden :

a) 4-[2-(4-Chlor-benzolsulfonamido)ethyl]nitrobenzol

Zu einer Mischung aus 9.0 g (44 mmol) 4-Nitrophenethylaminohydrochlorid (Chem. Ber. 45, 2431 (1921), 100 ml Dichlormethan und 13.5 g Triethylamin tropft man unter Eiskühlung die Lösung von 9.8 g (44 mmol) 4-Chlorbenzolsulfochlorid in 50 ml Dichlormethan. Man rührt 1 h nach, gießt auf Eis, extrahiert mit Dichlormethan, trocknet und engt ein. Es verbleiben 14.0 g Titelverbindung (89% d.Th.) vom Schmp. 151-152°C (aus Ethanol).

b) 4-[2-(4-Chlor-benzolsulfonamido)ethyl]anilin-hydrochlorid

11.5 g (34 mmol) der vorstehenden Nitroverbindung werden in 1 Liter Methanol über 0.5 g sulfidiertem Platinkatalysator hydrisiert. Nach Aufnahme der berechneten Wasserstoffmenge wird filtriert und mit etherischer Chlorwasserstofflösung versetzt. Man erhält 10.5 g Titelverbindung (quantitativ) vom Schmp. 228-230°C.

c) Brenztraubensäure-4-[2-(4-chlorbenzolsulfonamido)-ethyl]phenyl-hydrazon

Eine Mischung aus 8.9 g (26 mmol) des vorstehenden Hydrochlorids, 300 ml Wasser und 14 ml konz. Salzsäure wird bei Raumtemp. mit einer Lösung aus 1.8 g Natriumnitrat in 20 ml Wasser versetzt.

Zu einer Mischung aus 3.9 g (26 mmol) 2-Methylacetessigester und 100 g Eiswasser tropft man gleichzeitig die vorstehende Diazoninusalzlösung sowie eine Lösung von 14 g Kaliumhydroxid in 40 ml Wasser. Man rührt 15 min nach, stellt mit Salzsäure sauer, extrahiert mit Ether, trocknet und engt ein.

Es verbleiben 13.0 g (91% d.Th.) eines roten Öls, das roh weiterverarbeitet wird.

d) 5-[2-(4-Chlor-benzolsulfonamido)ethyl]indol-2-carbonsäureethylester

Eine Mischung aus 1.0 g (2.4 mmol) des vorstehenden Öls, 9 ml Eisessig und 1 ml konz. Schwefelsäure wird 5 min zum Sieden erhitzt. Man gießt auf Eis, extrahiert mit Essigester, wäscht neutral, trocknet und engt ein. Nach Umkristallisation aus Ethanol erhält man 300 mg (31% d.Th.) Titelverbindung vom Schmp. 192-195°C.

## Ansprüche

1. Sulfonamide der allgemeinen Formel I

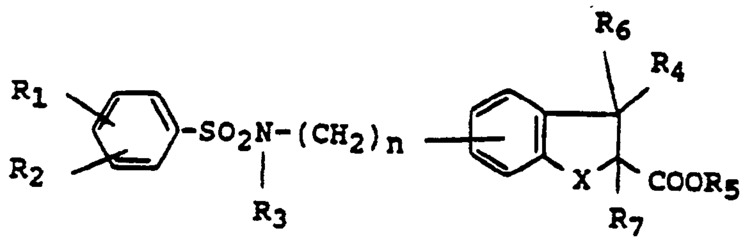

(I),

in welcher

$R_1$ und $R_2$ gleich oder verschieden sein können und jeweils Wasserstoff, ein Halogenatom, eine $C_1$- bis $C_6$-Alkyl-, $C_1$- bis $C_6$-Alkoxy-, $C_1$- bis $C_6$-Alkylthio-, $C_1$- bis $C_6$-Alkylsulfinyl-, $C^1$- bis $C_6$-Alkylsulfonyl-, Trifluormethyl-, Hydroxy-oder Cyanogruppe bedeuten,

$R_3$ und $R_8$ gleich oder verschieden sein können und jeweils Wasserstoff, eine $C_1$- bis $C_6$-Alkyl-, eine Benzyl- oder eine $C_1$- bis $C_6$-Alkanoylgruppe bedeuten,

$R_4$ und $R_5$ gleich oder verschieden sein können und jeweils Wasserstoff oder eine $C_1$- bis $C_6$-Alkylgruppe bedeuten,

$R_6$ und $R_7$ jeweils Wasserstoff darstellen oder gemeinsam eine Bindung bilden,

X für Sauerstoff, Schwefel, eine Gruppe NOH oder eine Gruppe $NR_8$ steht und

n eine ganze Zahl von 1 bis 4 bedeutet, deren physiologisch verträgliche Salze anorganischer oder organischer Basen.

2. Sulfonamide der Formel I gemäß Anspruch 1,

in welcher

$R_1$, $R_3$ und $R_8$ Wasserstoff

$R_2$ Wasserstoff, Brom, Chlor, Methyl oder Methoxy

$R_4$ Wasserstoff oder Methyl

$R_5$ Wasserstoff oder Ethyl bedeuten,

$R_6$ und $R_7$ gemeinsam eine Bindung bilden,

X für Sauerstoff, Schwefel oder eine Gruppe $NR_8$ steht,

n eine ganze Zahl 1 oder 2 bedeutet und der Sulfonamidorest in 5-Stellung des Heterocyclus steht.

3. Verfahren zur Herstellung von Sulfonamiden der Formel I

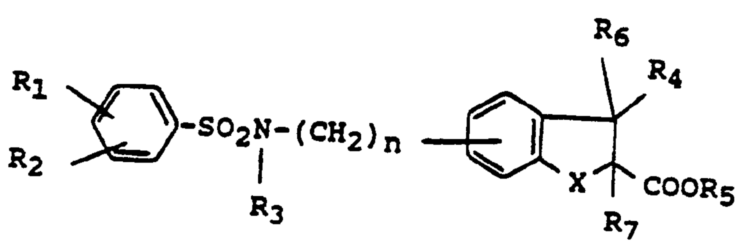

(I),

in welcher

$R_1$ und $R_2$ gleich oder verschieden sein können und jeweils Wasserstoff, ein Halogenatom, eine $C_1$- bis $C_6$-Alkyl-, $C_1$- bis $C_6$-Alkoxy-, $C_1$- bis $C_6$-Alkylthio-, $C_1$- bis $C_6$-Alkylsulfinyl-, $C_1$- bis $C_6$-Alkylsulfonyl-, Tri-

fluormethyl-, Hydroxy- oder Cyanogruppe bedeuten,

$R_3$ und $R_8$ gleich oder verschieden sein können und jeweils Wasserstoff, eine $C_1$- bis $C_6$-Alkyl-, eine Benzyl- oder eine $C_1$- bis $C_6$-Alkanoylgruppe beteuten,

$R_4$ und $R_5$ gleich oder verschieden sein können und jeweils Wasserstoff oder eine $C_1$- bis $C_6$-Alkylgruppe beteuten,

$R_6$ und $R_7$ jeweils Wasserstoff darstellen oder gemeinsam eine Bindung bilden,

X für Sauerstoff, Schwefel, eine Gruppe NOH oder eine Gruppe $NR_8$ steht und

n eine ganze Zahl von 1 bis 4 bedeutet, deren physiologisch verträgliche Salze anorganischer oder organischer Basen, dadurch gekennzeichnet, daß man in an sich bekannter Weise entweder

a) eine Verbindung der allgemeinen Formel II

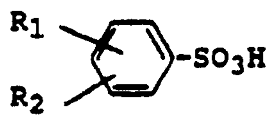

(II),

in welcher

$R_1$ und $R_2$ die obengenannte Bedeutung haben, oder einem reaktiven Derivat hiervon, mit einer Verbindung der allgemeinen Formel III

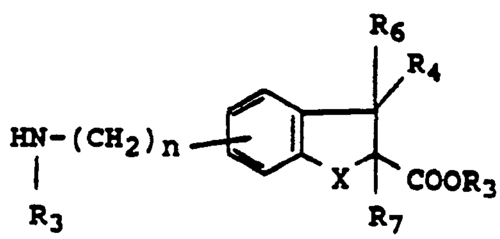

(III),

in welcher

$R_3$, $R_4$, $R_5$, $R_6$, $R_7$, X und n die obengenannte Bedeutung haben, oder

b) eine Verbindung der allgemeinen Formel IV

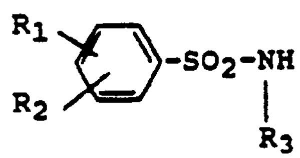

(IV),

in welcher

$R_1$, $R_2$ und $R_3$ die obengenannte Bedeutung haben, mit einer Verbindung der allgemeinen Formel V

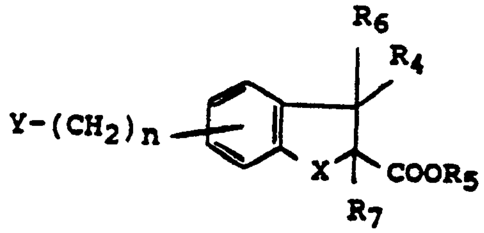

(V),

in welcher

$R_4$, $R_5$, $R_6$, $R_7$, X und n die obengenannte Bedeutung haben und Y einen reaktiven Rest darstellt, umsetzt, oder

c) für den Fall, daß X die Gruppe NH darstellt und $R_6$ und $R_7$ gemeinsam eine Bindung bilden, gemäß einer kombinierten Japp-Klingemann/Fischer-Indol-Synthese eine Verbindung der Formel VI

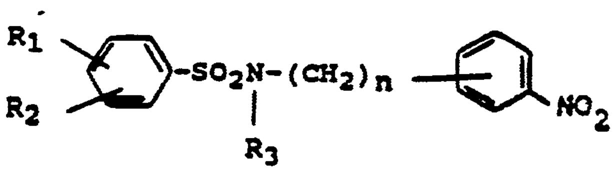

(VI)

in der $R_1$, $R_2$, $R_3$ und n die angegebene Bedeutung haben, reduziert, die entstandene Aminogruppe in üblicher Weise in eine Diazoniumgruppe überführt, die Diazoniumgruppe mit 2-Methylacetessigester umsetzt und das entstandene Hydrazon zum Indol cyclisiert, und anschließend gewünschtenfalls einen Rest R3, $R_5$ oder $R_8$ in einen anderen, durch die jeweilige Definition von $R_3$, $R_5$ oder $R_8$ gegebenen Rest umwandelt,
eine von $R_6$ und $R_7$ gemeinsam gebildete Bindung durch Hydrogenolyse aufhebt,
eine für $R_1$ oder $R_2$ stehende Alkylthiogruppe durch Oxidation in eine Alkylsulfinylgruppe oder Alkylsulfonylgruppe überführt,
eine für $R_1$ oder $R_2$ stehende Alkoxygruppe in eine Hydroxygruppe oder eine Hydroxygruppe in eine Alkoxygruppe umwandelt
und die erhaltenen Verbindungen der Formel I gewünschtenfalls durch Neutralisation mit nichttoxischen Basen in ihre physiologisch verträglichen Salze umwandelt.

4. Arzneimittel enthaltend eine Verbindung gemäß Anspruch 1 oder 2 neben üblichen Träger- und Hilfsstoffen.

5. Verwendung von Verbindungen gemäß Anspruch 1 oder 2 zur Herstellung von Arzneimitteln zur Behandlung von Stoffwechselerkrankungen.

6. Verwendung von Verbindungen gemäß Anspruch 1 oder 2 zur Herstellung von Arzneimitteln zur Behandlung von Durchblutungsstörungen.

**Claims**

1. Sulphonamides of the general formula I

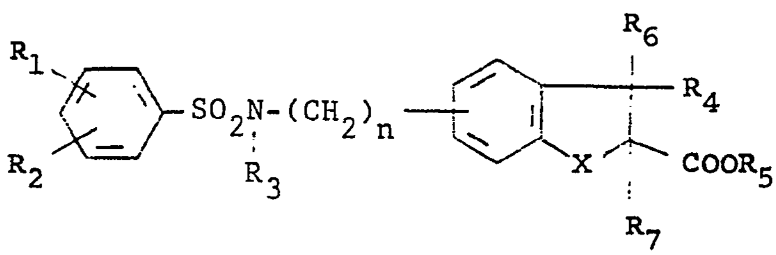

(I)

in which $R_1$ and $R_2$ can be the same or different and each signifies hydrogen, a halogen atom, a $C_1$ to $C_6$-alkyl, $C_1$ to $C_6$-alkoxy, $C_1$ to $C_6$-alkylthio, $C_1$ to $C_6$-alkylsulphinyl, $C_1$ to $C_6$-alkylsulphonyl, trifluoromethyl, hydroxyl or cyano group, $R_3$ and $R_8$ can be the same or different and each signifies hydrogen, a $C_1$ to $C_6$-alkyl, a benzyl or a $C_1$ to $C_6$-alkanoyl group, $R_4$ and $R_5$ can be the same or different and each signifies hydrogen or a $C_1$ to $C_6$-alkyl group, $R_3$ and $R_7$ each represent hydrogen atoms or together form a valency bond, X stands for oxygen, sulphur, a group NOH or a group $NR_8$ and n signifies a whole number from 1 to 4 ; their physiologically acceptable salts of inorganic or organic bases.

2. Sulphonamides of the formula I according to claim 1, in which $R_1$, $R_3$ and $R_8$ signify hydrogen, $R_2$ hydrogen, bromine, chlorine, methyl or methoxy, $R_4$ hydrogen or methyl, $R_5$ hydrogen or ethyl, $R_8$ and $R_7$ together form a valency bond, X stands for oxygen, sulphur atom or a group $NR_8$, n signifies a whole number 1 or 2 and the sulphonamido radical stands in the 5-position of the heterocycle.

3. Process for the preparation of sulphonamides of the formula I

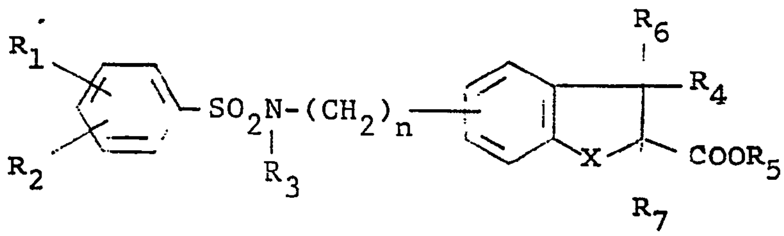

(I)

in which $R_1$ and $R_2$ can be the same or different and each signifies hydrogen, a halogen atom, a $C_1$ to $C_6$-alkyl, $C_1$ to $C_6$-alkoxy, $C_1$ to $C_6$-alkylthio, $C_1$ to $C_6$-alkylsulphinyl, $C_1$ to $C_6$-alkylsulphonyl, trifluoromethyl, hydroxyl or cyano groups, $R_3$ and $R_8$ can be the same or different and each signifies hydrogen, a $C_1$ to $C_6$-alkyl, a benzyl or a $C_1$ to $C_6$-alkanoyl group, $R_4$ and $R_5$ can be the same or different and each signifies hydrogen or a $C_1$ to $C_6$-alkyl group, $R_6$ and $R_7$ each represent hydrogen or together form a valency bond, X stands for oxygen, sulphur, a group NOH or a group $NR_8$ and n signifies a whole number from 1 to 4, of the physiologically acceptable salts of inorganic or organic bases, characterised in that, in per se known manner, one either

a) reacts a compound of the general formula II

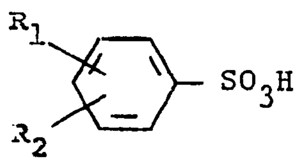

(II)

in which $R_1$ and $R_2$ have the above-given meanings, or a reactive derivative thereof with a compound of the general formula III

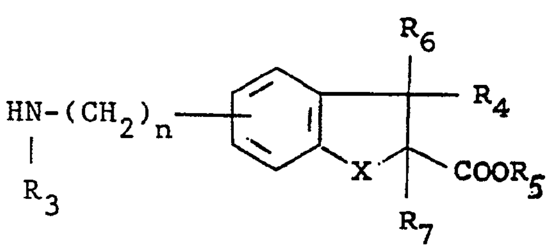

(III)

in which $R_3$, $R_4$, $R_5$, $R_6$, $R_7$ X and n have the abovegiven meanings, or

b) reacts a compound of the general formula IV

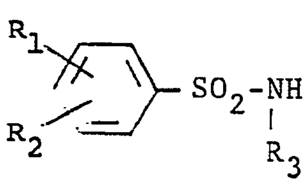

(IV)

in which $R_1$, $R_2$ and $R_3$ have the above-given meanings, with a compound of the general formula V

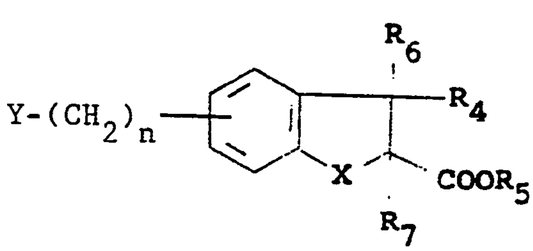

(V)

in which $R_4$,-$R_5$, $R_6$, $R_7$, X and n have the above-given meanings and Y represents a reactive residue, or

c) for the case that X represents the group NH and $R_6$ and $R_7$ together form a valency bond, reduces a compound of the general formula VI

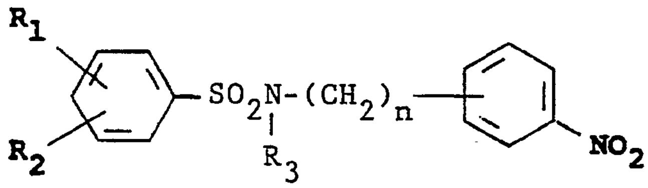

(VI)

in which $R_1$, $R_2$, $R_3$ and n have the above-given meanings, according to a combined Japp-Klingemann/Fischer indole synthesis, converts the resultant amino group in the usual way into a diazonium group, reacts the diazonium group with 2-methylacetoacetic acid ester and cyclises the resultant hydrazone to the indole, and subsequently, if desired, converts a radical $R_3$, $R_5$ or $R_8$ into another radical $R_3$, $R_5$ or $R_8$ given by the definition in question, removes a valency bond formed by $R_6$ and $R_7$ together by hydrogenolysis, converts an alkylthio group standing for $R_1$ or $R_2$ into an alkylsulphinyl group or alkylsulphonyl group by oxidation, converts an alkoxy radical standing for $R_1$ or $R_2$ into a hydroxyl group or a hydroxyl group into an alkoxy radical and, if desired, converts the compounds obtained of the formula I into their physiologically acceptable salts by neutralisation with non-toxic bases.

4. Medicaments containing a compound according to claim 1 or 2, as well as usual carrier and adjuvant materials.

5. Use of compounds according to claim 1 or 2 for the preparation of medicaments for the treatment of metabolic diseases.

6. Use of compounds according to claim 1 or 2 for the preparation of medicaments for the treatment of circulatory disturbances.

## Revendications

1. Sulfonamides de formule générale I

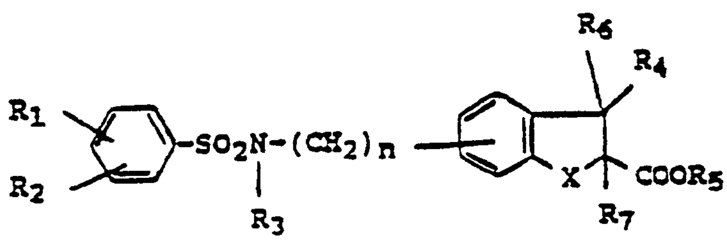

(I),

où

$R_1$ et $R_2$ peuvent être identiques ou différents et représentent chacun un atome d'hydrogène, un atome d'halogène, un groupe alkyle en $C_1$-$C_6$, alcoxy en $C_1$-$C_6$, alkylthio en $C_1$-$C_6$, alkylsulfinyle en $C_1$-$C_6$, alkylsulfonyle en $C_1$-$C_6$, trifluorométhyle, hydroxy ou cyano,

$R_3$ et $R_8$ peuvent être identiques ou différents et représentent chacun un atome d'hydrogène, un groupe alkyle en $C_1$-$C_6$, benzyle ou alcanoyle en $C_1$-$C_6$,

$R_4$ et $R_5$ peuvent être identiques ou différents et représentent chacun un atome d'hydrogène ou un groupe alkyle en $C_1$-$C_6$,

$R_6$ et $R_7$ représentent chacun un atome d'hydrogène ou forment en commun une liaison,

X représente un atome d'oxygène, un atome de soufre, un groupe NOH ou un groupe $NR_8$ et

n vaut un nombre entier de 1 à 4,

leurs sels formés avec des bases minérales ou organiques, physiologiquement acceptables.

2. Sulfonamides de formule générale I selon la revendication 1,

où

$R_1$, $R_3$ et $R_6$ représentent un atome d'hydrogène,

$R_2$ représente un atome d'hydrogène, de brome, de chlore, de méthyle ou de méthoxy,

$R_4$ représente un atome d'hydrogène ou un groupe méthyle,
$R_5$ représente un atome d'hydrogène ou un groupe éthyle,
$R_6$ et $R_7$ forment en commun une liaison,
X représente un atome d'oxygène, un atome de soufre ou un groupe $NR_8$,
n vaut un nombre entier de 1 ou 2 et
le reste sulfonamido est en position 5 de l'hétérocycle.

3. Procédé de préparation de sulfonamides de formule I

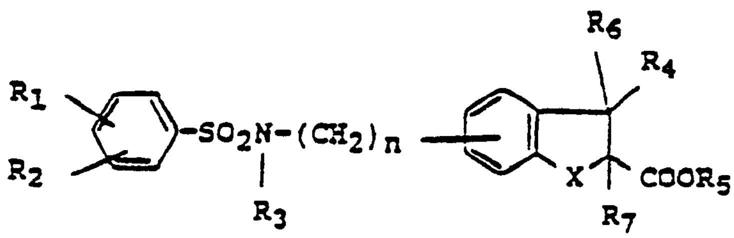

(I),

où

$R_1$ et $R_2$ peuvent être identiques ou différents et représentent chacun un atome d'hydrogène, un atome d'halogène, un groupe alkyle en $C_1$-$C_6$, alcoxy en $C_1$-$C_6$, alkylthio en $C_1$-$C_6$, alkylsulfinyle en $C_1$-$C_6$, alkylsulfonyle en $C_1$-$C_6$, trifluorométhyle, hydroxy ou cyano,
$R_3$ et $R_8$ peuvent être identiques ou différents et représentent chacun un atome d'hydrogène, un groupe alkyle en $C_1$-$C_6$, benzyle ou alcanoyle en $C_1$-$C_6$,
$R_4$ et $R_5$ peuvent être identiques ou différents et représentent chacun un atome d'hydrogène ou un groupe alkyle en $C_1$-$C_6$,
$R_6$ et $R_7$ représentent chacun un atome d'hydrogène ou forment en commun une liaison,
X représente un atome d'oxygène, un atome de soufre, un groupe NOH ou un groupe $NR_8$ et
n vaut un nombre entier de 1 à 4,
leurs sels formés avec des bases minérales ou organiques, physiologiquement acceptables.
caractérisé en ce que, d'une manière connue en soi, on fait réagir, soit
a) un composé de formule générale II

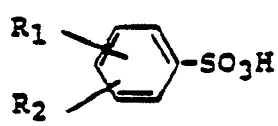

(II),

où

$R_1$ et $R_2$ ont les significations mentionnées ci-dessus ou un de leurs dérivés réactifs,
avec un composé de formule générale III

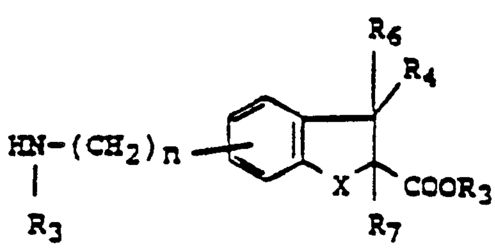

(III),

où

$R_3$, $R_4$, $R_5$, $R_6$, $R_7$, X et n ont les significations mentionnées ci-dessus, ou
b) un composé de formule générale IV

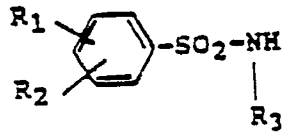

(IV),

où $R_1$, $R_2$ et $R_3$ ont les significations mentionnées ci-dessus, avec un composé de formule générale V

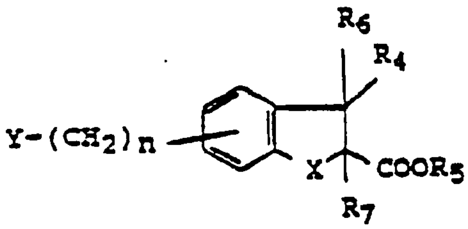

(V),

où $R_4$, $R_5$, $R_6$, $R_7$, X et n ont les significations mentionnées ci-dessus et Y représente un groupe réactif, ou

c) dans le cas où X représente le groupe NH et $R_6$ et $R_7$ forment en commun une liaison, on réduit, selon une synthèse combinée Japp-Klingemann/Fischer-indole, un composé de formule VI

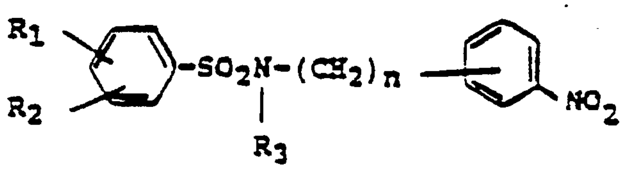

(VI)

où $R_1$, $R_2$, $R_3$ et n ont les significations mentionnées ci-dessus,

on transforme le groupe amino formé d'une manière usuelle en groupe diazonium, on fait réagir le groupe diazonium avec l'acétate de 2-méthyle et l'on cyclise l'hydrazone formée en indole,

et ensuite, si on le souhaite, on transforme le groupe $R_3$, $R_5$ ou $R_8$ en un autre groupe donné par la définition de chacun des groupes $R_3$, $R_5$ ou $R_8$,

on coupe par hydrogénolyse une liaison formée conjointement par $R_6$ et $R_7$,

on transforme un groupe alkylthio représenté par $R_1$ ou $R_2$, par oxydation, en un groupe alkyle sulfinyle ou alkylsulfonyle,

on transforme un groupe alcoxy représenté par $R_1$ ou $R_2$ en un groupe hydroxy, ou un groupe hydroxy en un groupe alcoxy,

et l'on transforme les composés de formule I obtenus éventuellement par neutralisation avec des bases non toxiques, en leurs sels physiologiquement acceptables.

4. Médicament comprenant un composé selon la revendication 1 ou 2, en plus de supports et adjuvants usuels.

5. Utilisation des composés selon la revendication 1 ou 2, pour la préparation de médicaments destinés au traitement des troubles du métabolisme.

6. Utilisation des composés selon la revendication 1 ou 2, pour la préparation de médicaments destinés au traitement des troubles de la circulation.